# EUROPEAN PATENT SPECIFICATION

(11) **EP 1 317 455 B1**
(45) Date of publication and mention of the grant of the patent: **04.08.2004**
(21) Application number: 01963665.3
(22) Date of filing: 05.09.2001
(51) Int. Cl.: C07D 471/04, C07D 213/803, C07D 213/82

(54) **PROCESS FOR PREPARING A SUBSTITUTED IMIDAZOPYRIDINE COMPOUND**
VERFAHREN ZUR HERSTELLUNG EINER SUBSTITUIERTEN IMIDAZOPYRIDINVERBINDUNG
PROCEDE DE PREPARATION D'UN COMPOSE D'IMIDAZOPYRIDINE SUBSTITUE

(30) Priority: 07.09.2000 SE 0003186
(43) Date of publication of application: 11.06.2003
(73) Proprietor: AstraZeneca AB, 151 85 Södertälje (SE)
(72) Inventor: ELMAN, Björn, S-195 34 Märsta (SE); ERBACK, Silke, CH-5742 Kölliken (CH); THIEMERMANN, Eric, CH-1700 Fribourg (CH)
(86) International application number: PCT/SE2001/001897
(87) International publication number: WO 2002/020523

(56) References cited:
- EP-A1- 0 033 094
- EP-A2- 0 308 917
- WO-A1-99/55706

## Description

### FIELD OF THE INVENTION

The present invention relates to a new process for the preparation of a substituted imidazopyridine compound, more specifically a new process for the preparation of a 2,3-dimethylimidazo[1,2-a]pyridine substituted in the 6-position by a carboxamido or a carboxyalkyl group. In further aspects, the present invention also relates to new intermediates used in the process.

### BACKGROUND AND PRIOR ART

The present invention relates to a new process suitable for large-scale preparation of a substituted imidazopyridine compound of formula (1), 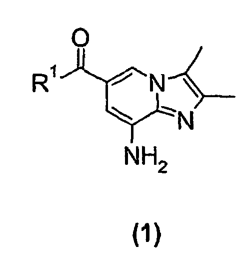 wherein R¹ is a C₁-C₆ alkoxy or NH₂ group, comprising the step of reacting a compound of the formula (2) 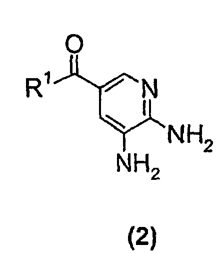 wherein R¹ is a C₁-C₆ alkoxy or NH₂ group, with a 3-halo-2-butanone compound in cyclohexanone.

A similar reaction is described in EP 33094, EP 204 285, EP 228 006, EP 308 917, and WO 99/55706 wherein a substituted aminopyridine compound of the general formula (X) 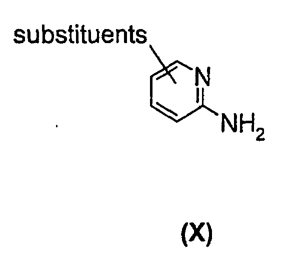 is reacted with a compound of formula 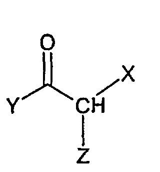 wherein X is i.a. H, CH₃ or an ester group, such as COOCH₃ or COOC₂H₅,
Y is i.a. CH₃, CH₂CH₃, and
Z is a leaving group, such as halogen, mesyl or tosyl,
to give a compound of the general structure 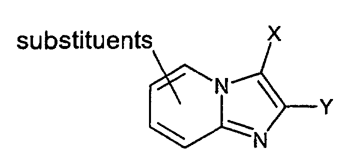 wherein X and Y are as described above.

The reaction is carried out in an inert solvent, such as acetone, alcohols, benzene, N,N-dimethylformamide, tetrahydrofurane, chloroform, or diethyl ether, preferably at elevated temperature, and optionally in the presence of an inorganic or organic base.

The reaction is characterized by long reaction times, e.g. 16 to 84 hours, high reaction temperatures and relatively low yields, e.g. 22% to 55%. The reaction is thereby not suitable for large-scale preparation of substituted imidazopyridine compounds.

We have surprisingly found that if the process of the present invention is carried out as described herein the reaction time can be shortened, the reaction temperature can be lowered and the yield is increased.

### OUTLINE OF THE INVENTION

The present invention provides a new process for large-scale preparation of substituted imidazopyridine compound of formula (1) 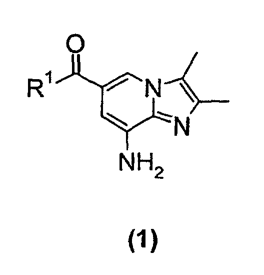 wherein R¹ is a C₁-C₆ alkoxy or NH₂ group, comprising the step of reacting a compound of the formula (2) 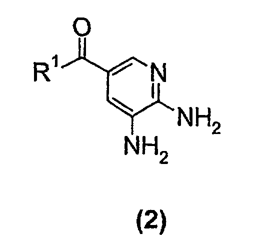 with a 3-halo-2-butanone compound in cyclohexanone.
In a first embodiment of the present invention a compound of the formula (2) 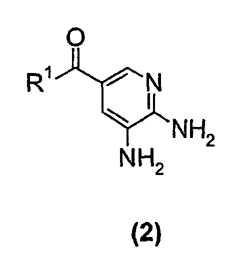 wherein R¹ is a C₁-C₆ alkoxy group, is reacted with a 3-halo-2-butanone compound in cyclohexanone to give a compound of the formula (1) 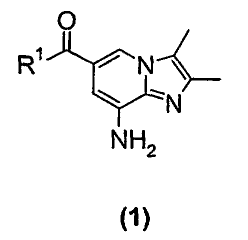 wherein R¹ is a C₁-C₆ alkoxy group.

In a second embodiment of the present invention a compound of the formula (2) 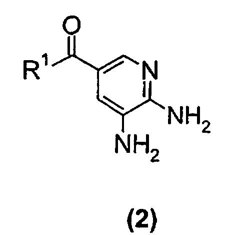 wherein R¹ is a NH₂ group, is reacted with a 3-halo-2-butanone compound in cyclohexanone to give a compound of the formula (1) 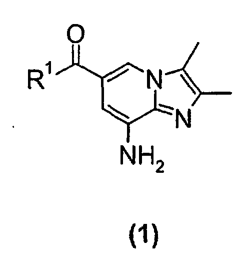 wherein R¹ is NH₂ group.

The process of the present invention is performed by solving or suspending a compound of formula (2) 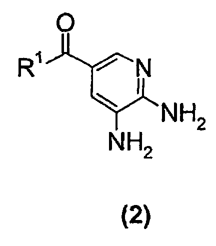 wherein R¹ is a C₁-C₆ alkoxy or NH₂ group, in cyclohexanone and adding a 3-halo-2-butanone compound, heat the reaction for a few hours and thereafter isolate a compound of formula (1) 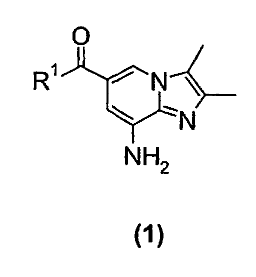 wherein R¹ is a C₁-C₆ alkoxy or NH₂ group, in high yields.

The amount of cyclohexanone is not crucial for carrying out the present invention, and can therefore in practical circumstances be adjusted according to needs and equipment used. It is also possible to mix cyclohexanone with inert solvents, such as ethers. Example of suitable inert solvents comprises, but is not limited to, to tetrahydrofuran (THF). The amount of inert solvent can be up to around 50%, by volume, without causing a decrease in yield.

The amount of 3-halo-2-butanone compound is not critical for carrying out the present invention. It is for practical and economical reasons preferred to add 1.1 to 5 molar equivalents, preferably 1.1 to 2 equivalents. Example of suitable 3-halo-2-butanone compounds comprises, but is not limited to, 3-bromo-2-butanone and 3-chloro-2-butanone, of which the latter is preferred.

Reaction temperatures and reaction times can be varied to meet the actual need. It is preferred to have a reaction temperature from 80°C to 100°C. This reaction temperature gives a complete reaction within a few hours, *e.g.* 1 to 4 hours. Conversion is usually above 95% and the isolated yield is usually above 70%.

The starting material to be used in the present invention can be prepared as disclosed in WO 99/55706 or alternatively as is described below in Scheme 1.

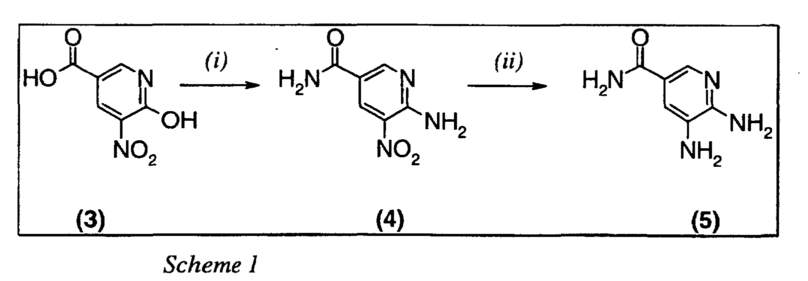

### Step i

Compound (3) in Scheme 1 is treated with thionyl chloride, or any equivalent reagent, at elevated temperature in an appropriate solvent for a few hours to give the corresponding chloride compound. The reaction is performed using around 1 to 5 equivalents thionyl chloride, preferably 1 to 2.5 equivalents, in toluene at approximately 100°C for 2 to 8 hours. The corresponding chloride compound is thereafter treated with 2 to 25 equivalents ammonia, preferably 3 to 12 equivalents, in the same solvent as above at approximately ambient temperature to give compound (4).

### Step ii

Compound (4) in Scheme 1 is hydrogenated in an aqueous alcoholic solution using a catalyst to give compound (5). Example of suitable catalyst comprises, but is not limited, to palladium, ruthenium or mixtures thereof. Pd-Ru/C paste is the preferred catalyst. Examples of alcohols comprises, but is not limited to, methanol, ethanol and propanol, of which methanol is preferred.

The substituted imidazopyridine compound of formula (1), 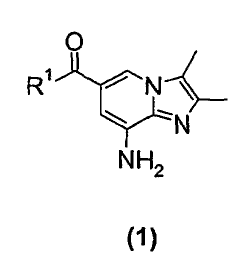 wherein R¹ is a C₁-C₆ alkoxy or NH₂ group, prepared according to the present invention can thereafter be used to prepare certain substituted imidazopyridine derivatives that are particularly effective as inhibitors of the gastrointestinal H⁺, K⁺-ATPase and thereby as inhibitors of gastric acid secretion.

Compounds of the Formula (1) can be reacted with a compound of the Formula (6) 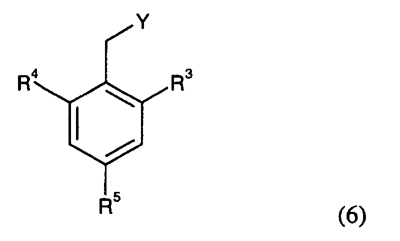 wherein R³ is H, C₁-C₆ alkyl, hydroxylated C₁-C₆ alkyl or halogen; R⁴ is H, C₁-C₆ alkyl, hydroxylated C₁-C₆ alkyl or halogen; R⁵ is H, or halogen; and Y is a leaving group, such as a halide, tosyl or mesyl group, to give a compound of Formula (7). 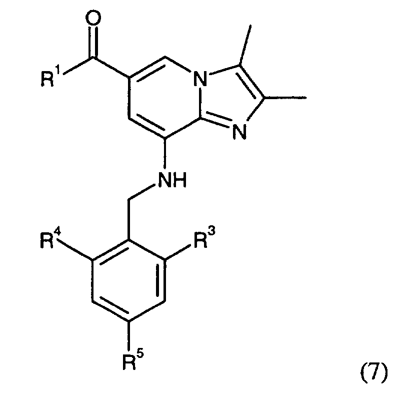 wherein R¹, R³, R⁴, and R⁵ are as defined above. It is convenient to conduct this reaction in an inert solvent, e.g. acetone, acetonitrile, dimethoxyethane, methanol, ethanol or dimethylformamide with or without a base. The base is e.g. an alkali metal hydroxide, such as sodium hydroxide and potassium hydroxide, an alkali metal carbonate, such as potassium carbonate and sodium carbonate; or an organic amine, such as triethylamine.

Compounds of the Formula (7) wherein R¹ is C₁-C₆ alkoxy can thereafter be further reacted with an amino compound of the general Formula (8) 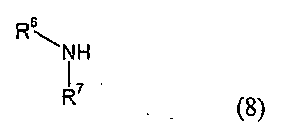 wherein R⁶ and R⁷ are the same or different and chosen from a group consisting of H, C₁-C₆ alkyl, hydroxylated C₁-C₆ alkyl, C₁-C₆ alkoxy-substituted C₁-C₆ alkyl, hydroxylated C₁-C₆ alkoxy-substituted C₁-C₆ alkyl, aryl, to give the corresponding amide compound.

R⁶ and R⁷ may together with the nitrogen atom to which they are attached, form a saturated or unsaturated ring optionally containing one or more further heteroatoms thereby forming e.g. morpholine, piperazine, pyrrolidine, or piperidine.

The reaction can be carried out by heating the reactants in the neat amino compound or dissolved in an inert solvent under standard conditions.

Alternatively can compounds of the Formula (7) 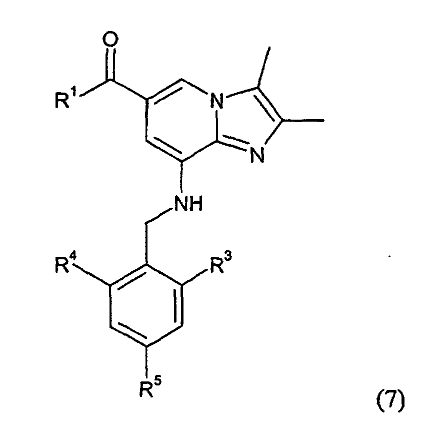 wherein R³, R⁴, and R⁵ are as defined above and R¹is an NH₂ group, be hydrolyzed under standard conditions to the corresponding carboxylic acid compounds of Formula (9) 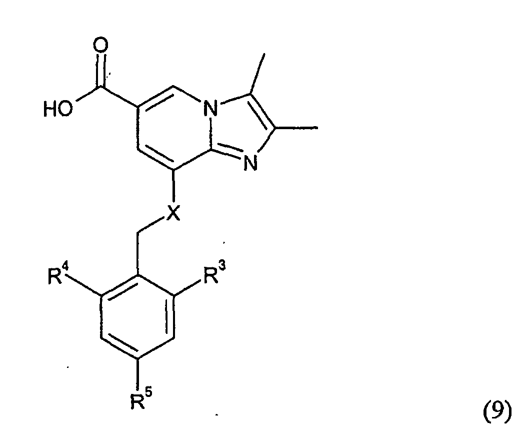 wherein R³, R⁴, and R⁵ are as defined above.

Compounds of the Formula (9) can thereafter be reacted with amino compounds of Formula (8) 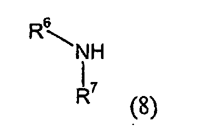 wherein R⁶ and R⁷ are as defined above, in the presence of a coupling reagent to give the corresponding amide compound. The reaction can be carried out in an inert solvent under standard conditions.

### EXAMPLES

### Example 1.1

### Preparation of Bromobutanone

In a reactor, sodium bromide (84 kg) is suspended in dimethylformamide (125 1). 3-Chloro-2-butanone (85 kg) is added at 15°C-30°C. Stirring is continued for 4 hours and then filtered: The filtercake is washed with cyclohexanone (38 1). The bromobutanone thereby prepared is ready to be used in the cyclisation step.

### Example 1.2

### Synthesis of methyl 8-amino-2,3-dimethylimidazo[1,2-a]pyridine-6-carboxylate

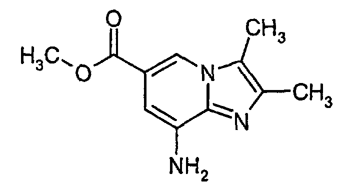

To a suspension of 5,6-diamino-nicotinic acid methyl ester (1 eq., 5.1 g) in cyclohexanone (50 ml) bromobutanone (1.2 equiv., 3.9 ml) was added over 10 min. The mixture was heated to 100°C (inner temperature) and stirred 2.5 h at this temperature. The mixture was cooled to room temperature and the pale solid was filtered off and was washed with TBME (3x10ml). Drying under reduced pressure at 45°C. Yield: 6.53 g (75%).

### Example 1.3

### Synthesis of ethyl 8-amino-2,3-dimethylimidazo[1,2-a]pyridine-6-carboxylate

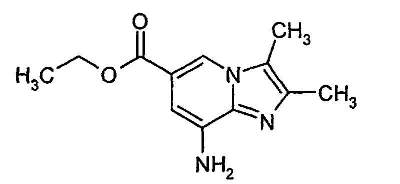

To a suspension of 5,6-diamino-nicotinic acid ethyl ester (1 eq., 5.0 g) in cyclohexanone (50 ml) bromobutanone (1.4 equiv., 5.95 g) was added over 15 min. The dark brown mixture was heated to 100°C (inner temperature) and stirred 1.5 h at this temperature. The mixture was cooled to room temperature and the light brown solid was filtered off and was washed with TBME (20 ml). Drying under reduced pressure at 45°C. Yield: 5.06 g (65%).

### Example 1.4

### Synthesis of isopropyl 8-amino-2,3-dimethylimidazo[1,2-a]pyridine-6-carboxylate

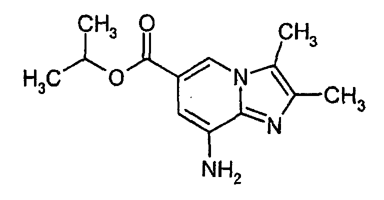

To a suspension of 5,6-diamino-nicotinic acid isopropyl ester (1 eq., 5.1 g) in cyclohexanone (50 ml) bromobutanone (1.2 equiv., 3.4 ml) was added over 10 min. The dark brown mixture was heated to 100°C (inner temperature) and stirred 1.5h at this temperature. The suspension was cooled to room temperature and the pale yellow solid was filtered off and was washed with TBME (3x10ml). Drying under reduced pressure at 45°C. Yield: 6.0 g (74%).

### Example 1.5

### Synthesis of 8-amino-2,3-dimethylimidazo[1,2-a]pyridine-6-carboxamide

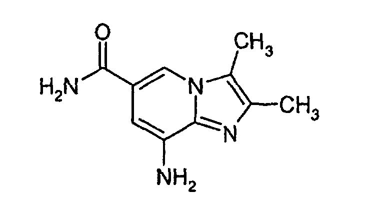

5,6-Diamino-nicotinamide (50 g, 0.313 mol (assay: 95.4%), 1.0 equiv.) was suspended in cyclohexanone (250 mL). The suspension was heated to 100°C. The filtrate (bromobutanone in cyclohexanone) was added at 100°C over 1 h 10 min. Heating was continued for 3 h and the heating source was thereafter removed. The reaction mixture was allowed to cool to 20°C and stirred at this temperature for another 2 h. The solid was filtered off, washed carefully with TBME (2 x 330 mL) and dried to yield 70.3 g of title compound. Yield: 70%.

### Example 1. 6

### Synthesis of 8-amino-2,3-dimethylimidazo[1,2-a]pyridine-6-carboxamide

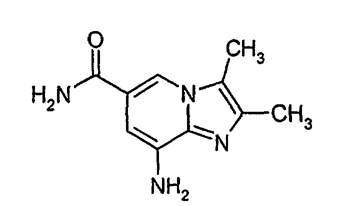

NaBr (27.0 g; 0.259 mol; 1.33 equiv) was suspended in cyclohexanone (220 mL) and 3-chloro-2-butanone (25.7 mL; 0.242 mol; 1.24 equiv) was added in one portion. The mixture was heated to 80°C and stirred for 3 h. The mixture was cooled to 50°C, the white solid was filtered off and washed with cyclohexanone (60 mL). 5,6-Diamino-nicotinamide (30 g; 0.1946 mol; 1.0 equiv) was added to the filtrate and the mixture was heated to 100°C for 4 h, after which 98% conversion was determined by HPLC. The reaction mixture was cooled to 20°C, stirring was continued for 2h at 20°C. The solid was filtered off, washed with TBME (220 mL) and dried to yield 46.6 g of the title compound. Yield: 73%.

### Example 1.7

### Synthesis of 8-amino-2,3-dimethylimidazo[1,2-a]pyridine-6-carboxamide

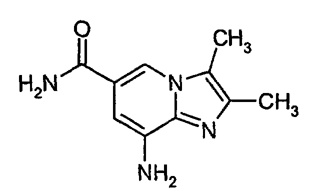

5,6-Diamino-nicotinamide (30.0 g; 0.183 mol; 1.0 equiv) was suspended in cyclohexanone (280 mL). 3-Bromo-2-butanone (24 mL; 0.22 mol; 1.2 equiv) was added and the mixture was heated for 4 h to 100°C.The reaction mixture was cooled to 20°C and allowed to stir for another 2 h. The solid was filtered off, washed with TBME (200 mL) and dried to yield 48.4 g of the title compound. Yield: 78%.

### Example 1.8

### Synthesis of methyl 2,3-dimethyl-8-(2,6-dimethylbenzylamino)-imidazo[1,2-a]pyridine-6-carboxylate

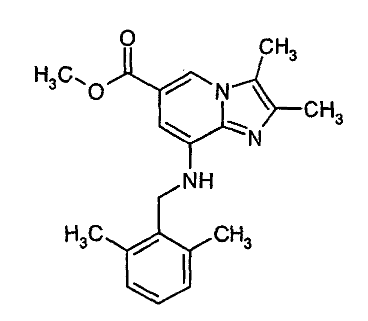

Methyl 8-amino-2,3-dimethylimidazo[1,2-a]pyridine-6-carboxylate (0.8 g, 3.6 mmol), 2,6-dimethylbenzylchloride (0.57 g, 3.7 mmol), sodium carbonate (1.0 g, 9.4 mmol) and a catalytic amount of potassium iodide were added to acetonitrile (10 ml) and were refluxed for 20 h. Following filtration, the salts were washed with methylene chloride and the solvents were evaporated under reduced pressure. The residue was purified by column chromatography on silica gel using methylene chloride: ethyl acetate (75:25) as eluent. The yellow residue was treated with hexane to give 0.23 g (19%) of the title product.

### Example 1.9

### Synthesis of ethyl 2,3-dimethyl-8-(2-ethyl-6-methylbenzylamino)-imidazo[1,2-a]pyridine-6-carboxylate

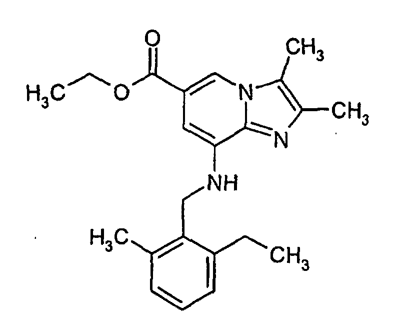

Ethyl 8-amino-2,3-dimethylimidazo[1,2-a]pyridine-6-carboxylate (0.7 g, 3.0 mmol), 2-ethyl-6-methylbenzylchloride (0.5 g, 3.0 mmol), sodium carbonate (0.64 g, 6.0 mmol) and a catalytic amount of potassium iodide were added to acetone (50 ml) and were refluxed for 20 h. Following filtration, the acetone was evaporated under reduced pressure to give an oil. The oily product was purified by column chromatography on silica gel using diethyl ether : petroleum ether (1:1) as eluent to give 0.12 g (9%) of the title product. ¹H-NMR (500 MHz, CDCl₃): δ 1.25 (t, 3H), 1.5 (t, 3H), 2.35 (s, 3H), 2.42 (s, 3H), 2.44 (s, 3H), 2.75 (q, 2H), 4.45-4.5 (m, 4H), 4.9 (bs, 1H), 6.8 (s, 1H), 7.05-7.2 (m, 3H), 8.1 (s, 1H)

### Example 1.10

### Synthesis of 2,3-dimethyl-8-(2-ethyl-6-methylbenzylamino)-N-propyl-imidazo[1,2-a]pyridine-6-carboxamide

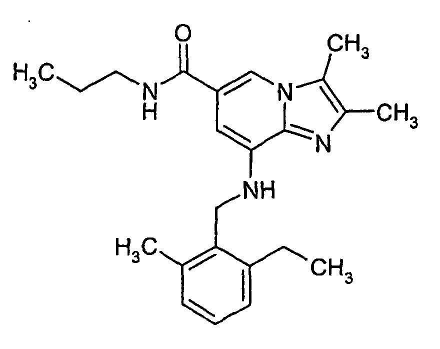

Ethyl 2,3-dimethyl-8-(2-ethyl-6-methylbenzylamino)-imidazo[1,2-a]pyridine-6-carboxylate (0.12 g, 0.33 mmol), propylamine (1.0 g, 17 mmol) and a catalytic amount of sodium cyanide were refluxed in methanol (20 ml) for 24 h. An additional amount of propylamine (1.0 g, 17 mmol) was added and the reaction mixture was refluxed for 24 h. The solvent was evaporated under reduced pressure and the residue was purified by column chromatography on silica gel using diethyl ether as eluent. Crystallization from diethyl ether gave 0.053 g (42%) of the title compound. ¹H-NMR (300 MHz, CDCl₃): δ 1.0 (t, 3H), 1.2 (t, 3H), 1.65-1.75 (m, 2H), 2.3 (s, 3H), 2.35 (s, 3H), 2.38 (s, 3H), 2.7 (q, 2H), 3.4-3.5 (m, 2H), 4.35 (d, 2H), 4.9 (bs, 1H), 6.2 (bs, 1H), 6.35 (s, 1H), 7.0-7.2 (m, 4H), 7.85 (s, 1H).

### Example 1.11

### Synthesis of 2,3-dimethyl-8-(2-ethyl-6-methylbenzylamino)-imidazo[1,2-a]pyridine-6-carboxamide

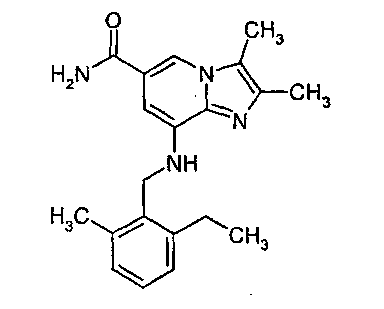

8-Amino-2,3-dimethylimidazo[1,2-a]pyridine-6-carboxamide (3.3 g, 16.2 mmol), 2-ethyl-6-methylbenzylchloride (2.73 g, 16.2 mmol), potassium carbonate (8.0 g, 58 mmol) and potassium iodide (1.1 g, 6.6 mmol) were added to acetone (150 ml) and refluxed for 20 h. An additional amount of 2-ethyl-6-methylbenzylchloride (1.0 g, 5.9 mmol) was added and the reaction mixture was refluxed for 7 h. Methylene chloride (60 ml) and methanol (30 ml) were added. The reaction mixture was filtered and the solvents were evaporated under reduced pressure. The residue was purified by column chromatography on silica gel using methylene chloride: methanol (100:7) as eluent. Crystallization from ethyl acetate gave 2.8 g (50%) of the title compound. ¹H-NMR (300 MHz, CDCl₃): δ 1.2 (t, 3H), 2.34 (s, 3H), 2.36 (s, 3H), 2.38 (s, 3H), 2.7 (q, 2H), 4.4 (d, 2H), 4.9 (bs, 1H), 6.0 (bs, 2H), 6.45 (s, 1H), 7.0-7.2 (m, 3H), 7.9, (s, 1H).

### Example 1.12

### Synthesis of 2,3-dimethyl-8-(2-ethyl-6-methylbenzylamino)-imidazo[1,2-a]pyridine-6-carboxylic acid

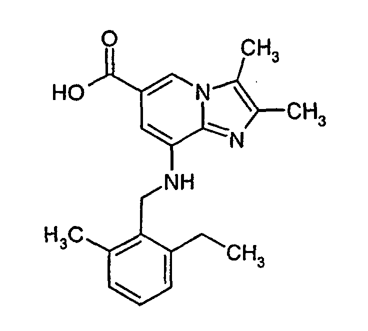

2,3-dimethyl-8-(2-ethyl-6-methylbenzylamino)-imidazo[1,2-a]pyridine-6-carboxamide mesylate (11.0 g, 0.025 mol) and sodium hydroxide (7.0 g, 0.17 mol) were solved in ethanol (95 %) (120 ml) and was refluxed for 20 h. The solvent was evaporated under reduced pressure and to the residue was added water (150 ml). The pH was adjusted to 5 by addition of conc. HCl and acetic acid and the solid that precipitated was isolated by filtration, washed with water and acetone, and dried to give 7.6 g (88 %) of the title compound. ¹H-NMR (500 MHz, DMSO-d₆): δ 1.15 (t, 3H), 2.26 (s, 3H), 2.34 (s, 3H), 2.39 (s, 3H), 2.69 (q, 2H), 4.38 (d, 2H), 5.2 (bs, 1H), 6.73 (s, 1H), 7.07-7.2 (m, 3H), 8.12 (s, 1H)

### Example 1.13

### Synthesis of 2,3-dimethyl-8-(2-ethyl-6-methylbenzylamino)-6-(morpholinocarbonyl)-imidazo[1,2-a]pyridine

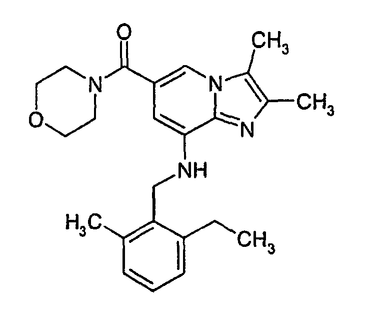

2,3-Dimethyl-8-(2-ethyl-6-methylbenzylamino)-imidazo[1,2-a]pyridine-6-carboxylic acid (0.15 g, 0.44 mmol) and o-Benzotriazol-1-yl-N,N,N',N'-Tetramethyluronium tetrafluoroborate (TBTU)(0.14 g, 0.44 mmol) were added to methylene chloride (10 ml). Morpholine (0.12 g, 1.4 mmol) was added and the reaction mixture was stirred at ambient temperature for 1.5 h. The reaction mixture was added to a column with silica gel and purification by chromatography using ethyl acetate : methylene chloride (1:1) as eluent gave 0.12 g (66%) of the desired product. ¹H-NMR (300 MHz, CDCl₃): δ 1.2 (t, 3H), 2.32 (s, 3H), 2.35 (s, 3H), 2.37 (s, 3H), 2.7 (q, 2H), 3.7 (s, 8H), 4.35 (d, 2H), 4.95 (bs, 1H), 6.15 (s, 1H), 7.0-7.2 (m, 3H), 7.4 (s, 1H)

### Example 1.14

### Synthesis of (2-ethyl-6 methylbenzylamino)-N(2-(2-hydroxyethoxy)ethyl)-2,3-dimethylimidazo[1,2-a]pyridine-6-carboxamide

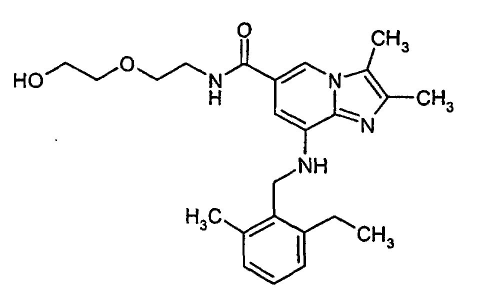

2,3-dimethyl-8-(2-ethyl-6-methylbenzylamino)-imidazo[1,2-a]pyridine-6-carboxylic acid (0.3 g, 0.88 mmol) and o-Benzotriazol-1-yl-N,N,N',N'-Tetramethyluronium tetrafluoroborate (TBTU)(0.29 g, 0.90 mmol) were added to methylene chloride (10 ml). 2-(2-aminoethoxy)ethanol (0.2 g, 1.9 mmol) was added and the reaction mixture was stirred at ambient temperature for 2 h. The solvent was evaporated under reduced pressure and the residue was purified by column chromatography on silica gel using methylene chloride:methanol (9:1) as eluent. Crystallization from diethyl ether gave 0.24 g (80%) of the desired product. ¹H-NMR (500 MHz, CDCl₃): δ 1.25 (t, 3H), 2.25 (s, 3H), 2.3 (s, 3H), 2.35 (s, 3H), 2.75 (q, 2H), 3.4-3.45 (m, 2H), 3.55-3.7 (m, 6H), 4.35 (d, 2H), 5.05 (t, 1H), 6.45 (s, 1H), 7.0-7.2 (m, 4H), 7.5 (s, 1H)

### Example 1.15

### Synthesis of isopropyl 8-[(2,6-dimethylbenzyl)amino]-2,3-dimethylimidazo[1,2-a]pyridine-6-carboxylate

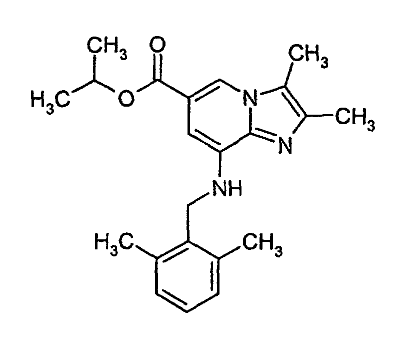

Isopropyl 8-amino-2,3-dimethylimidazo[1,2-a]pyridine-6-carboxylate (9.85 kg, 1.0 equiv., 29.71 mol) was suspended in isopropanol (59 L); NaI (0.6 equiv., 2.68 kg, 17.88 mol) and K₂CO₃ (2.5 equiv, 10.29 kg, 74.48 mol) were added and the mixture was heated to about 70°C. 2,6-Dimethylbenzyl chloride (1.1 equiv, 5.22 kg, 32.77 mol) was dissolved in isopropanol (-60 L) and this solution was added to the reaction mixture. After the addition was complete, the temperature was kept at 60°C for additional 1.5 hours. Additional K₂CO₃ was added (9.15 kg) and the resulting suspension was stirred for further 2h at 60°C. Additional 2,6-dimethylbenzyl chloride (2.76 kg) in isopropanol (22L) was added slowly at an temperature of 60°C; after the addition the reaction mixture was stirred for further 4 hours at this temperature. The suspension was diluted with water (124L), cooled, stirred and filtered. The filtercake was washed with water and then with cold isopropanol, dried under reduced pressure at 40°C to give 11.37 kg wet material, yield: 90%.

### Example 1.16

### Synthesis of 8-[(2,6-dimethylbenzyl)amino]-N-(2-hydroxyethyl)-2,3-dimethylimidazo[1,2-a]pyridine-6-carboxamide

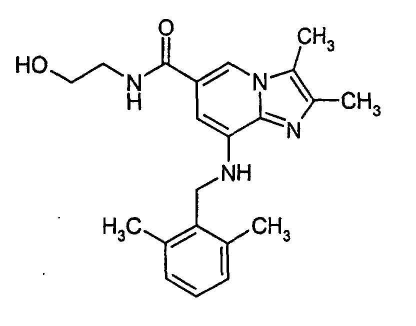

A reactor was charged with isopropyl 8-[(2,6-dimethylbenzyl)amino]-2,3-dimethylimidazo[1,2-a]pyridine-6-carboxylate (11.30 kg, 1 equiv., 27.02 mol) and THF (45 L), ethanolamine (18.97 kg, 11 equiv., 309.2 mol) was added at about 20°C. The suspension was heated to about 100°C. Some solvent was distilled off and then THF (35 L) was added and the distillation was continued. The procedure of adding THF and distilling it off was repeated until complete conversion. To the suspension ethanol (140L) was added and the suspension was heated to reflux. To obtain a clear solution additional ethanol (13L) was added. The hot solution was filtered and then cooled. The white solid was filtered off, washed with ethanol and dried to yield the product as a white powder. (8271 g).

### 2. PREPARATION OF STARTING MATERIALS

### Example 2.1

### Synthesis of 6-amino-5-nitro-nicotinamide

100 g of 6-hydroxy-5-nitro-nicotinic acid (0.54 mol; HPLC > 98% area) was suspended in toluene (750 mL). DMF (1 mL, 0.013 mol, 0.024 equiv.) was added and the mixture was heated to 110°C (inner temperature). Thionylchloride (99 mL, 2.5 equiv.) was added over 120 min. Heating was continued for 4h at 110°C. The reaction mixture was concentrated to half the volume (400 mL of solvent were distilled off), and toluene (400 mL) was added. This procedure was repeated once again (410 mL of toluene were distilled off and fresh toluene (410 mL) was added again). The solution was then cooled to 20°C and slowly added to aqueous ammonia (25%, 440 mL, 12 equiv.) over 40 min. Precipitation started immediately. During the addition the temperature was maintained below 15°C. After the addition had been completed the reaction mixture was allowed to warm up to room temperature and stirring was continued for 16h. The solid was filtered off, washed with water (500 mL), ethanol (250 mL), TBME (250 mL) and dried (50-10 mbar, 40°C bath temperature, 16 h) to yield 91.3 g of the title compound (0.501 mol, 87%).

### Example 2.2

### Synthesis of 5,6-diamino-nicotinamide

44.5 g of 6-amino-5-nitro-nicotinamide (0.24 mol; HPLC: 93% area) were suspended in methanol/water 1:1 (500 mL), 5.0 g of catalyst [Pd(4%)-Ru(1%)/C paste (62% H₂O type: 485; Johnson Matthey); type: 485; Johnson Matthey] was added. Hydrogenation was carried out at 5 bar and 30°C for 5h. After completion the catalyst was filtered off and washed with methanol/water 1/1 (50 mL). 480 mL of the solvent was distilled off. The resulting suspension was cooled to 20°C and filtered off. The solid was washed with methanol (20 mL) and TBME (30 mL). After drying (200-10 mbar; 40°C bath temperature, 16 h) 27.3 g of the title compound (0.18 mol, 73%) were obtained.

### Example 2.3

### Synthesis of 5,6-diamino-nicotinamide

42.3 g of 6-amino-5-nitro-nicotinamide (0.23 mol, HPLC: 93% area) was suspended in methanol/water 1:1 (500 mL). 5.2g of catalyst [Pd(5%)/C (57.8% H₂O); type: 39, Johnson Matthey] was added. Hydrogenation was carried out at 5 bar and 30°C for 4h. After completion the catalyst was filtered off and washed with methanol/water 1/1 (100 mL). 550 mL of the solvent was distilled off. The resulting suspension was cooled to 20°C and filtered off. The solid was washed with methanol (20 mL) and TBME (30 mL). After drying (200-10 mbar; 40°C bath temperature, 16 h) 28.5 g of the title compound (0.18 mol, 78%) was obtained.

## Claims

1. A process for preparing a substituted imidazopyridine compound of formula (1) 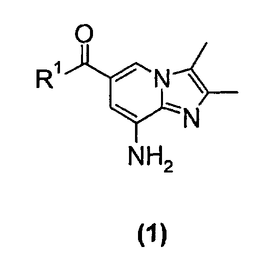 wherein R¹ is C₁-C₆ alkoxy or NH₂,
comprising the step of reacting a compound of the formula (2) 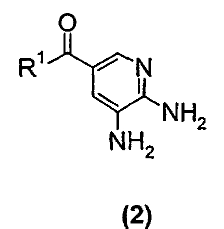 with a 3-halo-2-butanone compound in cyclohexanone.

2. A process according to claim 1, wherein the 3-halo-2-butanone compound is 3-bromo-2-butanone or 3-chloro-2-butanone.

3. A process according to claim 1 or 2, wherein the amount of the 3-halo-2-butanone compound is 1.1 to 5 molar equivalents.

4. A process according to claim 1, wherein the reaction temperature is 80°C to 100°C.

5. A process according to claim 1, wherein cyclohexanone is diluted with an inert solvent.

6. A process according to claim 1, wherein R¹ is C₁-C₆ alkoxy.

7. A process according to claim 1, wherein R¹ is NH₂.

8. A process according to claim 1, **characterized in that** compound (2) is prepared by a process comprising the step of hydrogenating a compound of formula (4) 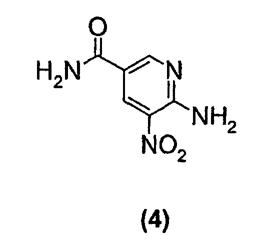 in an aqueous alcoholic solution using a catalyst.

9. A process according to claim 8, wherein the catalyst is a Pd-Ru/C paste.

10. A process according to any of claim 8 or 9, **characterized in that** compound (4) is prepared by a process comprising the step of reacting a compound of formula (3) 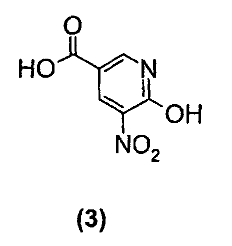 with thionyl chloride to give the corresponding chloride compound, which is thereafter treated with ammonia.

## Patentansprüche

1. Verfahren zur Herstellung von geläutertem Pyromellitsäureanhydrid, welches umfaßt:
Auflösen von roher Pyromellitsäure, die Trimellitsäure und Methyltrimellitsäure aus Nebenprodukten enthält, in Wasser,
dann Abkühlen der so erhaltenen wäßrigen Lösung, um eine Kristallisation als Pyromellitsäure durchzuführen, .
dann Abtrennen der so erhaltenen Kristalle aus dem Wasser,
dann Dehydratisieren der so abgetrennten Kristalle von Pyromellitsäure unter Erwärmen zur Erzeugung von Pyromellitsäureanhydrid,
dann Destillieren des so erzeugten Pyromellitsäureanhydrids in einem einstufigen Verfahren unter einem reduzierten Druck von 20 kPa (150 Torr) unter darunter bei einer Temperatur von 250 bis 400°C,
Abkühlen des so erhaltenen Dampfes von Pyromellitsäureanhydrid und
dadurch Gewinnen von geläuterten Kristallen von Pyromellitsäureanhydrid mit einem niedrigeren Gehalt von Trimellitsäure und Methyltrimellitsäure aus Nebenprodukten als die rohe Pyromellitsäure.

2. Verfahren gemäß Anspruch 1, worin das Verhältnis von Wasser zu roher Pyromellitsäure 2/1 bis 10/1 ist.

3. Verfahren gemäß Anspruch 1, worin die rohe Pyromellitsäure in Wasser bei einer Temperatur von 70 bis 180°C aufgelöst wird.

4. Verfahren gemäß Anspruch 1, worin die Lösung unter reduziertem Druck abgekühlt wird, um die Kristallisation der Pyromellitsäure durchzuführen.

5. Verfahren gemäß Anspruch 1, worin die Kristalle von Pyromellitsäure vom Wasser durch Filtration, Dekantieren oder Zentrifugentrennung abgetrennt werden.

6. Verfahren zur Herstellung von geläutertem Pyromellitsäureanhydrid, welches umfaßt:
Auflösen von roher Pyromellitsäure, die Trimellitsäure und Methyltrimellitsäure aus Nebenprodukten enthält, in Wasser in einem Verhältnis von Wasser zu roher Pyromellitsäure von 2/10 bis 10/1,
dann Durchführen einer Kontaktbehandlung der so erhaltenen wäßrigen Lösung mit Aktivkohle,
dann Abtrennen der wäßrigen Lösung von der Aktivkohle,
dann Abkühlen der wäßrigen Lösung zur Durchführung einer Kristallisation als Pyromellitsäure,
dann Abtrennen der so erhaltenen Kristalle von Pyromellitsäure vom Wasser,
dann Dehydratisieren der so erhaltenen Kristalle von Pyromellitsäure unter Erwärmen auf eine Temperatur von 170 bis 260°C,
dann Destillieren des so erzeugten geläuterten Pyromellitsäureanhydrids in einem einstufigen Verfahren unter reduziertem Druck von 20 kPa (150 Torr) oder darunter bei einer Temperatur von 250 bis 400°C,
Abkühlen des so erhaltenen Dampfes von Pyromellitsäureanhydrid und
dadurch Gewinnen von geläuterten Kristallen von Pyromellitsäureanhydrid mit einem niedrigeren Gehalt von Trimellitsäure und Methyltrimellitsäure aus Nebenprodukten als die rohe Pyromellitsäure.

7. Verfahren gemäß Anspruch 6, worin die Menge von Aktivkohle 1 bis 30 Gew.Teile auf 100 Gew.Teile von roher Pyromellitsäure ist.

8. Verfahren gemäß Anspruch 6, worin die rohe Pyromellitsäure in Wasser bei einer Temperatur von 70 bis 180°C aufgelöst wird.

9. Verfahren gemäß Anspruch 6, worin die Lösung unter reduziertem Druck abgekühlt wird, um die Kristallisation als Pyromellitsäure durchzuführen.

10. Verfahren gemäß Anspruch 6, worin die Kristalle von Pyromellitsäure vom Wasser durch Filtration, Dekantieren oder Zentrifugentrennung abgetrennt werden.

## Revendications

1. Procédé de production d'un anhydride pyromellitique, lequel procédé comprend les étapes qui consistent à :
dissoudre dans l'eau de l'acide pyromellitique brut qui contient comme sous-produits de l'acide trimellitique et de l'acide méthyltrimellitique,
refroidir ensuite la solution aqueuse ainsi obtenue pour réaliser la cristallisation sous la forme d'acide pyromellitique,
séparer alors de l'eau, les cristaux ainsi obtenus,
déshydrater alors par chauffage les cristaux d'acide pyromellitique ainsi obtenus, pour produire de l'anhydride pyromellitique,
distiller alors l'anhydride pyromellitique ainsi obtenu, dans un processus en une étape, sous pression réduite de 20k Pa (150 Torr) ou moins, à une température de 250 à 400°C,
refroidir les vapeurs d'anhydride pyromellitique ainsi obtenues et
recueillir ainsi des cristaux affinés d'anhydride pyromellitique dont la teneur en les sous-produits acide trimellitique et acide méthyltrimellitique est moindre que dans l'acide pyromellitique brut.

2. Procédé selon la revendication 1, dans lequel le rapport entre l'eau et l'acide pyromellitique brut est compris entre 2/1 et 10/1.

3. Procédé selon la revendication 1, dans lequel l'acide pyromellitique brut est dissous dans l'eau à une température de 70 à 180°C.

4. Procédé selon la revendication 1, dans lequel la solution est refroidie sous pression réduite, pour réaliser la cristallisation sous la forme d'acide pyromellitique.

5. Procédé selon la revendication 1, dans lequel les cristaux d'acide pyromellitique sont séparés de l'eau par filtration, décantation ou séparation par centrifugation.

6. Procédé de production d'anhydride pyromellitique affiné, lequel procédé comprend les étapes qui consistent à :
dissoudre dans l'eau de l'acide pyromellitique brut qui contient comme sous-produits de l'acide trimellitique et de l'acide méthyltrimellitique, dans un rapport entre l'eau et l'acide pyromellitique brut compris entre 2/1 et 10/1,
traiter alors par contact avec du charbon actif, la solution aqueuse ainsi obtenue,
séparer alors la solution aqueuse du charbon actif,
refroidir alors la solution aqueuse pour produire la cristallisation sous la forme d'acide pyromellitique,
séparer alors de l'eau les cristaux d'acide pyromellitique ainsi obtenus,
déshydrater alors par chauffage à une température de 170 à 260°C les cristaux d'acide pyromellitique ainsi obtenus,
distiller alors l'anhydride pyromellitique affiné ainsi obtenu, dans un processus en une étape, sous pression réduite de 20 kPa (150 Torr) ou moins, et à une température de 250 à 400°C,
refroidir les vapeurs d'anhydride pyromellitique ainsi obtenues, et
recueillir ainsi des cristaux affinés d'anhydride pyromellitique dont la teneur en les sous-produits acide trimellitique et acide méthyltrimellitique est moindre que dans l'acide pyromellitique brut.

7. Procédé selon la revendication 6, dans lequel la quantité de charbon actif est de 1 à 30 parties en poids pour 100 parties en poids d'acide pyromellitique brut.

8. Procédé selon la revendication 6, dans lequel l'acide pyromellitique brut est dissous dans l'eau à une température de 70 à 180°C.

9. Procédé selon la revendication 6, dans lequel la solution est refroidie sous pression réduite, pour réaliser la cristallisation sous la forme d'acide pyromellitique.

10. Procédé selon la revendication 6, dans lequel dans lequel les cristaux d'acide pyromellitique sont séparés de l'eau par filtration, décantation ou séparation par centrifugation.
